# EUROPEAN PATENT APPLICATION

(11) **EP 3 875 473 A1**
(43) Date of publication of application: **08.09.2021**
(21) Application number: 19824384.2
(22) Date of filing: 29.10.2019
(51) Int. Cl.: C07K 14/47, C07K 1/14, G01N 33/574

(54) **PROCESS FOR EXTRACTING, CONCENTRATING AND PURIFYING AN ANTIGEN FROM A BIOLOGICAL SAMPLE, COMPOSITION AND USES THEREOF**

(30) Priority: 29.10.2018 PT 2018115115
(71) Applicant: Universidade de Aveiro, 3810-193 Aveiro (PT)
(72) Inventor: FREIRE MARTINS, Mara Guadalupe, 3810-696 Aveiro (PT); MENDONÇA PEREIRA, Matheus, 3810-182 Aveiro (PT); ALMEIDA SOUSA, Ana Catarina, 6200-505 Covilhã (PT); COSTA ARAÚJO PEREIRA COUTINHO, João Manuel, 3830-008 Ílhavo (PT)
(74) Representative: Patentree
(86) International application number: PCT/IB2019/059277
(87) International publication number: WO 2020/089797

(57) **Abstract**

The present disclosure refers to the development of a new method of pre-treatment of biological samples, preferably from human urine, comprising the simultaneous extraction, concentration and purification of the prostate-specific antigen (PSA), a biomarker for prostate cancer. In this method, aqueous biphasic systems are used, composed of ionic liquids and salts which allow the selective extraction of PSA and its isoforms, in particular the benign prostate-specific antigen (bPSA) which is a specific molecular form of PSA found in the transition zone of patients suffering from benign prostatic hyperplasia, to the ionic liquid-enriched phase, and its simultaneous purification and concentration.

## Description

### TECHNICAL FIELD

The present disclosure refers to the development of a new method of pre-treatment of biological samples, preferably from human urine, comprising the simultaneous extraction, concentration and purification of the prostate-specific antigen (PSA), a biomarker for prostate cancer. In this method, aqueous biphasic systems are used, composed of ionic liquids and salts which allow the selective extraction of PSA and its isoforms, in particular the benign prostate-specific antigen (bPSA) which is a specific molecular form of PSA found in the transition zone of patients suffering from benign prostatic hyperplasia, to the ionic liquid-enriched phase, and its simultaneous purification and concentration.

This new method, by concentrating the biomarker of interest between 10 - 1000-fold, allows to overcome one of the great limitations associated with the use of biomarkers which lies in their low concentration in biologic fluids and consequent difficulty in its quantification. Therefore, by purifying and concentrating the biomarker, this method allows the posterior quantification of PSA and/or bPSA to be performed by routine analytical techniques, which are simpler, cheaper an available in less sophisticated laboratories. On the other hand, this method, while allowing the selective extraction of PSA and also its isoforms, allows to increase the specificity of this diagnostic technique, enhancing its predictive value and advances in differential diagnosis.

### Background art

Prostate cancer (PCa) is the 2^{nd} most common type of cancer in male subjects and the 1^{st} cause of death at a worldwide level, according to GLOBOCAN estimations reviewed in the article by Ferley et al. (International Journal of Cancer, 2015, 136, E359-E386) entitled "Cancer incidence and mortality worldwide: Sources, methods and major patterns in GLOBOCAN 2012*".*

The costs associated with the treatment of PCa were evaluated by Luengo-Fernandez et al. (The Lancet Oncology, 2013, 14, 1165-1174) in the article entitled *"Economic burden of cancer across the European Union: a population-based cost analysis*"*,* estimating that only for Europe these costs overtake 8,43 billion euros. In order to reduce the costs associated with PCa, integrated strategies of early diagnosis are fundamental.

The prostate cancer diagnosis is traditionally performed invasively by the doctor by digital rectal examination, trans-rectal ultrasound, cystoscopy and biopsy, or more recently and less invasively using biomarkers.

Biomarkers present huge advantages when compared to traditional methods, since they do not require direct clinical intervention on the client, there only being necessary to collect a biological sample, normally blood or urine. Nevertheless, there is a significant drawback associated with biomarkers, since the quantification thereof is hampered by the fact that there are low concentrations in the biological samples and because these are complex, requiring that previous treatment of the samples is made before it is possible to proceed to the quantification of the biomarker of interest.

The prostate-specific antigen (PSA) is a protein that has abnormal values in subjects suffering from prostate cancer (PCa) and is therefore used as biomarker in early diagnosis. Due to its strong correlation with PCa, the PSA blood test is most used in the early diagnosis of this type of cancer. There are several techniques available for the detection of PSA, namely, immunoassays, including ELISA, fluorescence microscopy, plasmon surface resonance, immunochromatography and immunoluminescence.

In clinical practice, immunoassays are mostly used since they are validated by the FDA (Food and Drug Administration, USA). Even though many of these techniques present a suitable detection limit, the majority present significant related drawbacks. The ELISA immunoassay, for instance, is a robust technique that allows the antigen identification; however, it requires processing by highly specialized technicians, it requires several laborious steps and it requires the use of specific antibodies which brings about a considerable increase in costs. On the other hand, it is necessary to label the target substance in the fluorescence and electrochemical methods for signal amplification. Moreover, the extended exposure of dyes to the excitation light source causes the bleaching thereof and the signal decrease, which may result in a sub estimation of the results.

In the last years, several alternative techniques have yet been reported, such as the use of DNA microarray technologies or proteins in the detection and quantification of PSA. These new alternatives are very promising in terms of detection limits; however, the use of this type of highly sophisticated technology in clinical practice is still far from being a reality. Mass spectrometry-based methods have also been proposed, with and without the use of antibodies. However, again, their use is conditioned by the fact that they require highly sophisticated equipment and unavailable in most clinical la boratories.

Besides being in the blood, PSA is present in different biological fluids, including urine. In fact, and according to the work "Urinary PSA: a potential useful marker when serum PSA is between 2.5 ng/mL and 10 ng/mL" published by Bolduc et al. (Canadian Urological Association Journal, 2007, 1, 377-381), the PSA quantification in urine is advantageous when the serum values are between 4.0 and 10.0 ng/mL, the so-called grey zone, for which the differential diagnosis between PCa and benign prostatic hyperplasia is difficult. In urine, the limit value of 150 ng/mL was established; this value exhibits a sensitivity of 92.5% and it is recommendable that patients with PSA levels <150 ng/mL perform a prostatic biopsy. However, despite the advantages of monitoring the urinary PSA levels, this technique is still not used in clinical practice since there are no simple and expedite alternatives that allow the detection and quantification thereof.

The pre-treatment of urine samples is not simple. There are several methods of extraction and pre-concentration of urine samples, namely, dialysis, (ultra)filtration, precipitation, ultracentrifugation and solid phase extraction. However, these methods are time-consuming, require several steps, often specific equipment, and generally carry protein losses which may compromise the biomarker quantification. Dialysis-based methods are often used to remove salts from urine, which is then concentrated using specific concentration techniques; however, this method is very time-consuming, the extraction and concentration requiring long periods of time. On the other hand, the solid phase extraction is generally faster, requires the acquisition of specific columns and the use of particular equipment which are not available in many laboratories. Moreover, there is a possibility of contamination by the used column.

Facing all these limitations, there is a need for the development of an alternative method of urine treatment that allows to eliminate the contaminating metabolites (e.g., other proteins, DNA, RNA) and that simultaneously concentrates PSA so that its quantification may be performed by simpler equipment that are available for instance in clinical laboratories of under developed and developing countries, where the prostate cancer-associated mortality is high, and where early diagnosis is particularly important.

The use of aqueous biphasic systems (ABS) is an alternative to the above described methods for PSA separation and concentration. Fedotoff et al. (Journal of Biomolecular Structure and Dynamics, 2012, 29, 1051-1064), used ABS composed of dextran-ficoll for PSA extraction; however, this method did not allow the complete PSA extraction, with partition coefficients of only 1,5 being achieved. Apart from this incomplete extraction, the authors found that the PSA partition to the dextran-rich phase decreased in the presence of other proteins. Electrostatic forces induced by protein-protein interactions lead to the formation of protein complexes, responsible for changes in the biomarker partition. The same team proposed an alternative method also based in ABS, not to evaluate the total PSA but to evaluate its isoforms, patent document US9354229B2, entitled "*Systems and methods for characterization of molecules*"*.* The method presented by the authors presupposes that each isoform, depending on its structure, migrates preferentially to each of the ABS phases. Therefore, after quantifying both phases, it is possible to calculate the K index which allows to categorize the PSA isoform mixture between benign and cancer phenotypes. Recently, this method was validated by Klein *et al.* in a prospective multicentre study published in 2017 in the journal "European Urology" (72, 942-949) and it is commercially available (http://www.cleveland-diagnostics.com/isopsa/). The method is relatively simple, involving the partition of different isoforms by mixture of the system, followed by centrifugation and determination of the K index after quantification of both phases. However, it does not extract contaminating proteins and implies the PSA quantification by immunoassays (ELISA), a great drawback that the present disclosure is able to overcome.

The known solutions in the state of the art illustrate the technical problem to be solved by the present disclosure.

### General description

This disclosure refers to the development of an alternative method of pre-treatment of biological samples based in aqueous biphasic systems (ABS) which comprise the use of ionic liquids (ILs) and salts to simultaneously extract, concentrate and purify the tumoral biomarker named prostate-specific antigen (PSA), and its varied isoforms, in particular its bPSA isoform.

In an embodiment, the biological sample to which the present disclosure refers to is urine.

In clinical practice, the quantification of the prostate-specific antigen (PSA) in the blood (serum) is considered as the main early stage diagnostic mean of prostate cancer (PCa). However, this process implies the blood collection, which is an invasive matrix. On the other hand, most of the available techniques for PSA quantification require an extensive sampling processing and sophisticated technical equipment to allow the detection of the low levels of this biomarker, which normally are not available in underdeveloped and developing countries where the prostate cancer-associated mortality is higher.

Considering that PSA may also be detected in urine, the present disclosure proposes a simple and economical method for the simultaneous extraction, purification and concentration of PSA and/or its isoforms from urine samples using aqueous biphasic systems (ABS) which comprise the use of ionic liquids (ILs) and salts.

With this disclosure, the pre-treatment of urine samples is performed, allowing that PSA and/or its isoforms may be completely extracted to the ionic liquid-enriched phase in a single step, without protein losses and with high concentration factors (CF). This disclosure thus refers to a pre-treatment in a single step, for that using the formed ABS only with salt, ionic liquid and urine, which represents an advantage in comparison to the currently used immunoassay and solid phase extraction systems. These systems involve several steps, which are laborious and expensive, and require highly sophisticated equipment for the PSA quantification. With this disclosure, it is possible to concentrate PSA between 10 - 1000-fold and, therefore, its quantification may be performed by laboratory equipment with higher detection limits, which are more accessible and less expensive. Furthermore, by being possible to identify bPSA in the same analysis, this disclosure contributes to an increase in the diagnostic specificity, enhancing its predictive value, additionally allowing advances in differential diagnosis. In general, this disclosure may be considered as an alternative and more economical pre-treatment platform for the simultaneous extraction, purification and concentration of cancer biomarkers from non-invasive samples (urine).

The main advantage of these ABS with ILs, as opposed to other already described ABS for the extraction and concentration of PSA, lies in the possibility of being able to manipulate the polarities of the coexisting phases in order to reach selective and complete extractions. The use of ionic liquids for the pre-treatment of urine samples for evaluation of other biomarkers has already been described, however, the used methods are different from the one we propose herein. In patent document CN 104569224B, imidazolium-based ionic liquids were used for the selective separation and consecutive concentration of different species of mercapto acid. However, it was necessary to add an organic solvent, as opposed to the present disclosure. Moreover, the quantification method used was liquid chromatography coupled with mass spectrometry (LC-MS/MS). In the present disclosure, this type of highly sophisticated equipment is not necessary since high concentration factors are accomplished and this way the PSA quantification may be performed in simpler and routine equipment.

One of the aims of the present disclosure was the development of a new pre-treatment platform of urine samples, a non-invasive biological matrix, allowing the simultaneous extraction, purification and concentration of tumoral biomarkers using aqueous biphasic systems composed of ionic liquids and salts.

In this way, the present disclosure refers to a process for extracting, concentrating and purifying a prostate-specific antigen (PSA) and benign prostate-specific antigen (bPSA) from a biological sample, in particular collected in a non-invasive way, comprising the following steps:
extracting the prostate-specific antigen (PSA) and benign prostate-specific antigen (bPSA) by preparing and homogenizing an aqueous biphasic solution comprising the biological sample, an ionic liquid and a salt;
concentrating said homogenized solution; and
purifying the prostate-specific antigen (PSA) and benign prostate-specific antigen (bPSA) of said concentrated solution.

In an embodiment, the proposed ionic liquids present buffer features, avoiding the need of using a salt with buffer ability, and are biocompatible. These two features are essential for the total extraction, that is, without losses of proteins, such as PSA and bPSA.

In an embodiment, the proposed ionic liquids are synthetized by acid-base reactions between the imidazolium hydroxides, respective quaternary ammoniums or quaternary phosphoniums, and the protonated forms of CHES, MES, TES, HEPES or Tricine (commonly known as *Good's buffers*), as described in the example presented by Taha et al. (Green Chemistry, 2014, 16, 3149-3159).

In an embodiment, the ionic liquid may have a mass ratio between 10-60 % (m_{ionic liquid}/m_{aqueous biphasic solution}), preferably has a mass ratio of 20-50 % (m_{ionic liquid}/m_{aqueous biphasic solution}), even more preferably has a mass ratio of 30% (m_{ionic liquid}/m_{aqueous biphasic solution}).

In an embodiment, the ionic liquids may be selected from the following list: ionic liquids of the imidazolium family, quaternary ammoniums and quaternary phosphoniums, with variable alkyl side chain size and presence of specific functional groups, combined with organic or inorganic anions.

In an embodiment, the ionic liquid may be selected from the following list: tetrabutylphosphonium cyclohexyl-2-aminoethanesulfonate [P₄₄₄₄][CHES], tetrabutylphosphonium 2-(N-morpholino)ethanesulfonate ([P₄₄₄₄][MES]), tetrabutylphosphonium 2-[[1,3-dihydroxy-2-(hydroxymethyl)propan-2-yl]amino]ethanesulfonate ([P₄₄₄₄][TES]), tetrabutylphosphonium 2-[4-(2-hydroxyethyl)piperazin-1-yl]ethanesulfonate ([P₄₄₄₄][HEPES]) or tetrabutylphosphonium N-2(2-hydroxy-1,1-bis(hydroxymethyl)ethyl)glycine ([P₄₄₄₄][Tricine]).

In an embodiment, and in order to obtain even better results, the ionic liquid is tetrabutylphosphonium cyclohexyl-2-aminoethanesulfonate [P₄₄₄₄][CHES].

In an embodiment, the salt may have a mass ratio between 5-50 % (mₛₐₗₜ/_{maqueous biphasic solution}), preferably has a mass ratio of 10-45 % (mₛₐₗt/m_{aqueous biphasic solution}), even more preferably has a mass ratio of 40% (m_{salt/}m_{aqueous biphasic solution}).

In an embodiment, the salt may be selected from the following list: sodium salt combined with organic or inorganic anions or potassium salt combined with organic or inorganic anions, preferably the salt may be potassium citrate (K₃C₆H₅O₇) or sodium citrate (Na₃C₆H₅O₇).

In an embodiment, and in order to obtain even better results, the salt may be potassium citrate (K₃C₆H₅O₇).

In an embodiment, and in order to obtain even better results, the ionic liquid may be tetrabutylphosphonium cyclohexyl-2-aminoethanesulfonate ([P₄₄₄₄][CHES]) and the salt may be potassium citrate (K₃C₆H₅O₇).

In an embodiment, the concentrating step comprises concentrating the prostate-specific antigen and benign prostate-specific antigen (bPSA) 10-1000-fold relative to the concentration of the prostate-specific antigen and benign prostate-specific antigen (bPSA) in the biological sample, preferably urine.

In an embodiment, the step of concentrating the prostate-specific antigen and benign prostate-specific antigen (bPSA) is performed by centrifugation, preferably at 187.80 - 18780 xg.

In an embodiment, the purifying step is performed by removal/extraction of the prostate-specific antigen (PSA) and benign prostate-specific antigen (bPSA) to an ionic liquid-enriched phase obtained after the purifying step.

In an embodiment, the biological sample may have a mass ratio between 5-40 % (m_{biological sample}/m_{aqueous biphasic solution}), preferably has a mass ratio of 10-35 % (m_{biological sample}/m_{aqueous biphasic solution}), even more preferably has a mass ratio of 30% (mb_{iological sample}/m_{aqueous biphasic solution}).

In an embodiment, the aqueous biphasic solution may have the following salt: ionic liquid: biological sample mass ratio of 40:30:30 (mₛₐₗ:m_{ionic liquid}:m_{biological sample}).

In an embodiment, the biological sample may be urine, preferably human urine.

In an embodiment, the purifying step may be performed between 1-60 minutes, preferably between 10-50 min, even more preferably between 20-40 min.

In an embodiment, the purifying step may occur at a temperature between 5-50 °C, preferably between 10-30 °C, even more preferably between 18-25 °C.

In an embodiment, the extracting step may be performed by means of a magnetic stirrer.

The present disclosure also refers to a composition comprising a biological sample, an ionic liquid and a salt, for use in medicine.

The present disclosure also refers to a composition comprising a biological sample, an ionic liquid and a salt, for use in an "in vivo" diagnostic method of the prostate cancer disease.

In an embodiment, the ionic liquid comprised in the composition for use in an "in vivo" diagnostic method of the prostate cancer disease now disclosed may have a mass ratio between 10-60 % (m_{ionic liquid/}m_{aqueous biphasic solution}), preferably has a mass ratio of 20-50 % (m_{ionic liquid/}m_{aqueous biphasic solution}), even more preferably has a mass ratio of 30% (m_{ionic liquid/}m_{aqueous biphasic solution}).

In an embodiment, the ionic liquid comprised in the composition for use in an "in vivo" diagnostic method of the prostate cancer disease now disclosed may be selected from the following list: tetrabutylphosphonium cyclohexyl-2-aminoethanesulfonate [P₄₄₄₄]_{[}CHES], tetrabutylphosphonium 2-(N-morpholino)ethanesulfonate ([P₄₄₄₄][MES]), tetrabutylphosphonium 2-[[1,3-dihydroxy-2-(hydroxymethyl)propan-2-yl]amino]ethanesulfonate ([P₄₄₄₄][TES]), tetrabutylphosphonium 2-[4-(2-hydroxyethyl)piperazin-1-yl]ethanesulfonate ([P₄₄₄₄][HEPES]) or tetrabutylphosphonium N-2(2-hydroxy-1,1-bis(hydroxymethyl)ethyl)glycine ([P₄₄₄₄][Tricine]), preferably wherein the ionic liquid is tetrabutylphosphonium cyclohexyl-2-aminoethanesulfonate ([P₄₄₄₄][CHES]).

In an embodiment, the salt comprised in the composition for use in an "in vivo" diagnostic method of the prostate cancer disease now disclosed, may have a mass ratio between 5-50 % (m_{salt/}m_{aqueous biphasic solution}), preferably has a mass ratio of 10-45 % (m_{salt/}m_{aqueous biphasic solution}), even more preferably has a mass ratio of 40% (mₛₐₗₜ/m_{aqueous biphasic solution}).

In an embodiment, the salt comprised in the composition for use in an "in vivo" diagnostic method of the prostate cancer disease now disclosed, may be potassium citrate (K₃C₆H₅O₇) or sodium citrate (Na₃C₆H₅O₇), preferably and in order to obtain better results it may be potassium citrate (K₃C₆H₅O₇).

In an embodiment, and in order to obtain even better results, the composition for use in an "in vivo" diagnostic method of the prostate cancer disease now disclosed may comprise the tetrabutylphosphonium cyclohexyl-2-aminoethanesulfonate ([P₄₄₄₄][CHES]) ionic liquid and the potassium citrate salt (K₃C₆H₅O₇).

In an embodiment, the biological sample comprised in the composition for use in an "in vivo" diagnostic method of the prostate cancer disease now disclosed, may have a mass ratio between 5-40 % (m_{biological sample}/m_{aqueous biphasic solution}), preferably has a mass ratio of 10-35 % (m_{biological sample}/m_{aqueous biphasic solution}), even more preferably has a mass ratio of 30% (mb_{iological sample/}m_{aqueous biphasic solution}).

In an embodiment, the biological sample is urine, preferably is human urine.

In an embodiment, and in order to obtain even better results, the composition for use in an "in vivo" diagnostic method of the prostate cancer disease now disclosed, may comprise a salt:ionic liquid:biological sample mass ratio of 30%:40%:30% (mₛₐₗₜ:m_{ionic liquid}:m_{biological sample}), being that when the biphasic aqueous solution comprised K₃C₆H₅O₇:[P₄₄₄₄][CHES]:urine in a 40%:30%:30% mass ratio, the biomarker extraction efficiency was 100%, but with lower protein losses, and as such it was chosen for future assays. However, other mass ratios, namely 40%:30%:40%, and other ionic liquids were also tested with results that are also positive in terms of biomarker extraction efficiency and with neglectable losses thereof during the process now disclosed.

The present disclosure also refers to a kit comprising a composition for use in an "in vivo" diagnostic method of the prostate cancer disease now disclosed.

### Brief description of the drawings

For an easier understanding of the present disclosure, drawings are herein attached, which represent preferential embodiments which however, do not intend to limit the object of the present disclosure.
**Figure 1****:** Schematic representation of the process of extraction and concentration of tumoral biomarkers of prostate cancer from human urine using aqueous biphasic systems composed of ionic liquids and salts.
**Figure 2****:** Phase diagrams of the ternary systems (ABS) composed of: IL + K3C6H5O7 + H2O at 25 °C: [P4444][Tricine] ( 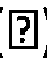 ); [P4444][HEPES] ( 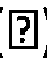 ); [P4444][TES] ( 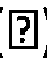 ); [P4444][MES] ( 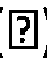 ); [P4444][CHES] ( 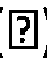 ); and adjusted (-) by the equation: [IL] = *Aexp*[(*B*[_{*sal*t}]^{0.5}*)* - (*C*[*sal*t]³)]*.*
**Figure 3****:** Chromatographic profiles obtained by size exclusion high-performance liquid chromatography (SE-HPLC) of the ionic liquid-enriched top phase and of the salt-enriched bottom phase of the systems composed of IL + K3C6H5O7 + H2O/PSA. For comparison purposes, the chromatographic profile of a pure PSA solution (50ng/L) is also represented.
**Figure 4****:** Schematic representation of the lever rule for the systems composed of IL + K3C6H5O7 + H2O at 25 °C: [P4444][Tricine] (A); [P4444][HEPES] (B); [P4444][TES] (C); [P4444][MES] (D); [P4444][CHES] (E); and adjusted (-) by the equation: [IL] = *Aexp*[(*B*[*sal*t]^{0.5}) - (*C*[*sal*t]³)].
**Figure 5****:** 3D Structure of PSA highlighting the catalytic triad (A) and results of molecular docking of PSA with the following ions: [P_{4444]}⁺ (B), [CHES]⁻ (C), and [MES]⁻ (D).

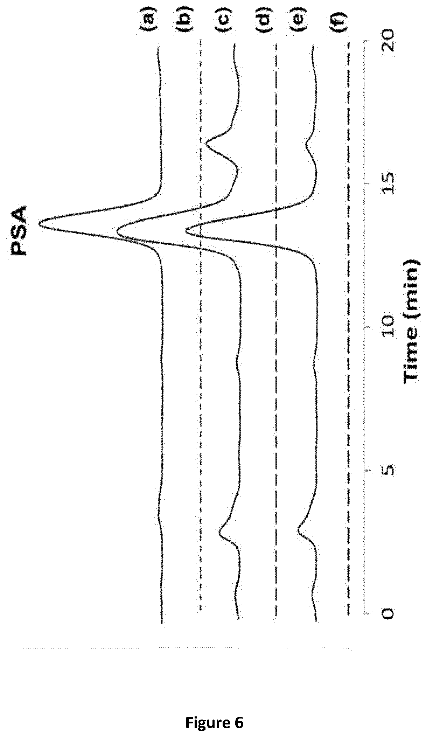

**Figure 6****:** Chromatographic profile obtained by size exclusion high-performance liquid chromatography (SE-HPLC): (a) pure PSA in aqueous solution (150 ng/mL); (b) human urine of a healthy male donor without the pre-treatment method described in the present disclosure; diagrams c-f correspond to samples of urine treated with the pre-treatment method described in the present disclosure: (c) ionic liquid-rich phase (pure urine); (d) salt-rich phase (pure urine); (e) ionic liquid-rich phase (urine with the addition of 150 ng/mL PSA); (f) salt-rich phase of the urine sample with the addition of 150 ng/mL PSA).

### DETAILED DESCRIPTION

The present disclosure refers to a pre-treatment method of non-invasive samples, namely urine, for the extraction and concentration of PSA, a biomarker for prostate cancer.

The advantages presented by this process compared to the existing traditional methods are the following. This process is a process that uses samples collected in a non-invasive way, since the use of human urine as matrix renders the process less invasive when compared to traditional methods that use human serum as matrix, which implies blood collection. This collection must be made by a healthcare professional, which increases the costs associated with the analysis. On the other hand, blood collection may be an unpleasant experience for some patients, diminishing the adherence to early diagnosis. This process is also characterized by its ease of use. The disclosure described herein is easy to use, it just requires adding a specific amount of urine to a pre-defined amount of ionic liquid and salt. To obtain the concentrated PSA solution, it is enough to shake vigorously and separate the phases, a vortex and a centrifuge only being needed.

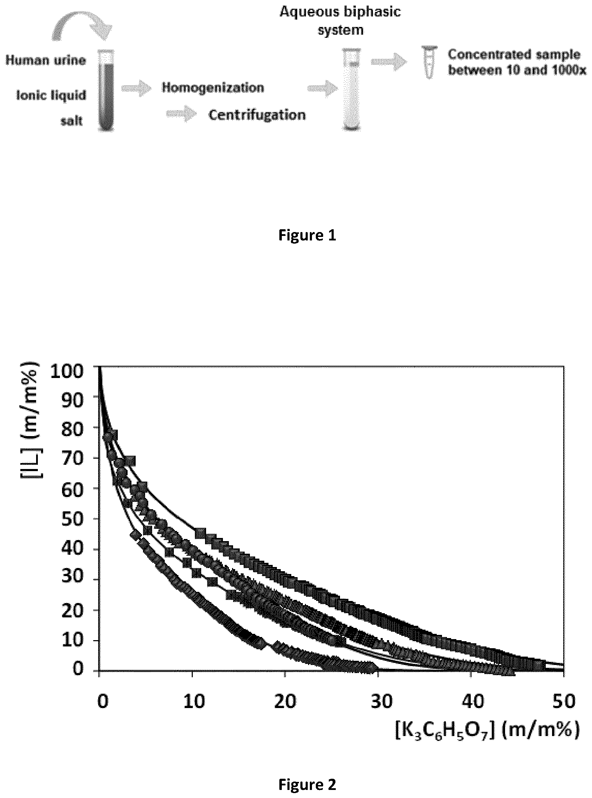

In one embodiment, in detail, the process of selective extraction and concentration of PSA from human urine may be performed in 4 sequential steps, such as illustrated in **figure 1****.** In the first step, preparation of the extraction system is proceeded, in the second step homogenization of the phases is proceeded, and in the third and fourth step the separation and recuperation of the phases for posterior analysis of PSA and its isoforms.

In an embodiment, the preparation of the extraction system is performed in the following manner: the first urine of the day may be collected to a sterile container and validated for clinical analysis. The sample volume must not be less than 30 mL. The sample should be immediately preserved at 4 °C until the procedure described in the present disclosure is performed.

In an embodiment, the extraction procedure is initiated after adding the urine with a pre-established amount of ionic liquid and salt. The amount of each component to be used is calculated based on the solubility curve and equilibrium lines of the phase diagrams of each IL/salt systems under consideration.

In an embodiment, homogenization may be performed as follows. The mixture may be homogenized in a vortex for 5 to 10 minutes.

In an embodiment, after homogenization, the sample is concentrated, for instance by means of centrifugation, for 1 to 60 minutes at a rotation speed between 187.8-18 780 xg and, for instance, at a temperature between 5 and 50 °C. In the end of the centrifugation, two phases are obtained, being that the ionic liquid-enriched phase is the top phase and the salt-rich phase is the bottom phase. The concentrated PSA solution is in the ionic liquid-rich phase. Thus, the concentrated PSA solution may be easily removed and the biomarker quantification may be performed in different equipment. However, since high concentration factors are obtained, this equipment does not need to be very sophisticated, neither to present low detection limits.

In an embodiment, the method described in the present disclosure comprises the addition of urine to a mixture containing an ionic liquid (IL) and a salt in order to form an aqueous biphasic system (ABS), which will allow the simultaneous selective extraction and concentration of PSA and its isoforms to the ionic liquid-enriched phase. Said mixture comprises the following mass ratio: salt:ionic liquid:urine, 40%:30%:30%.

In an embodiment, the method described in the present disclosure may be summarized in 4 steps. The first step comprises the preparation of the pre-treatment system of urine samples, namely weighing of each of the necessary components to form an ABS (urine, IL and salt). The amounts of each of the components to be used is determined based in equilibrium lines of phase diagrams of each of the ternary systems under consideration. In the second step homogenization of the components is performed by, for instance, vigorous stirring. In the third step centrifugation for phase separation is performed, followed by a fourth step which may comprise the analysis of concentrated PSA that is in the ionic liquid-enriched phase.

In an embodiment, the method described in the present disclosure was applied to real urine samples, being verified that PSA is in the top phase, IL-enriched phase, with no PSA being present in the opposed phase.

In an embodiment, the method described in the present disclosure was applied to several ABS composed of different ionic liquids, namely [P₄₄₄₄][CHES], tetrabutylphosphonium 2-(N-morpholino)ethanesulfonate ([P₄₄₄₄][MES]), tetrabutylphosphonium 2-[[1,3-dihydroxy-2-(hydroxymethyl)propan-2-yl]amino]ethanesulfonate ([P₄₄₄₄][TES]), tetrabutylphosphonium 2-[4-(2-hydroxyethyl)piperazin-1-yl]ethanesulfonate ([P₄₄₄₄][HEPES]), and tetrabutylphosphonium N-2(2-hydroxy-1,1-bis(hydroxymethyl)ethyl)glycine ([P₄₄₄₄][Tricine]).

In an embodiment, for the same concentration of K₃C₆H₅O₇ salt (10 (m/m)%), the formation ability of ABS varies as follows: [P₄₄₄₄][CHES] > [P₄₄₄₄][MES] > [P_{4444][}HEPES] ≈[P₄₄₄₄][TES] > [P₄₄₄₄][Tricine], as demonstrated in **figure 2****.**

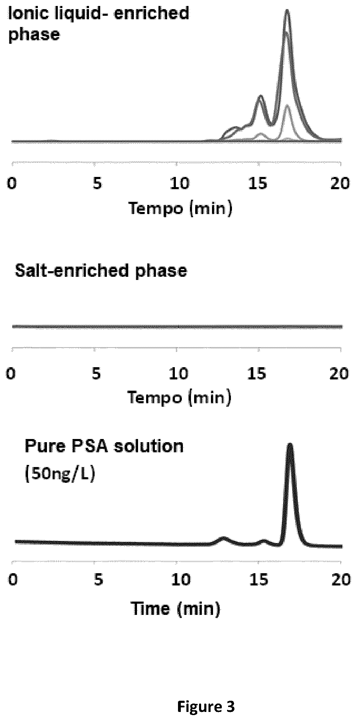

In an embodiment, the extraction efficiency for PSA to the ionic liquid-rich phase was 100% for all ABS tested as evidenced in **table 1.** The peak intensity of PSA in the IL-rich phase and consequently the PSA concentration, decreases as follows: [P₄₄₄₄][CHES] > [P₄₄₄₄][MES] > [P₄₄₄₄][TES]> [P₄₄₄₄][HEPES] > [P₄₄₄₄][Tricine], as demonstrated in **figure 3****.**

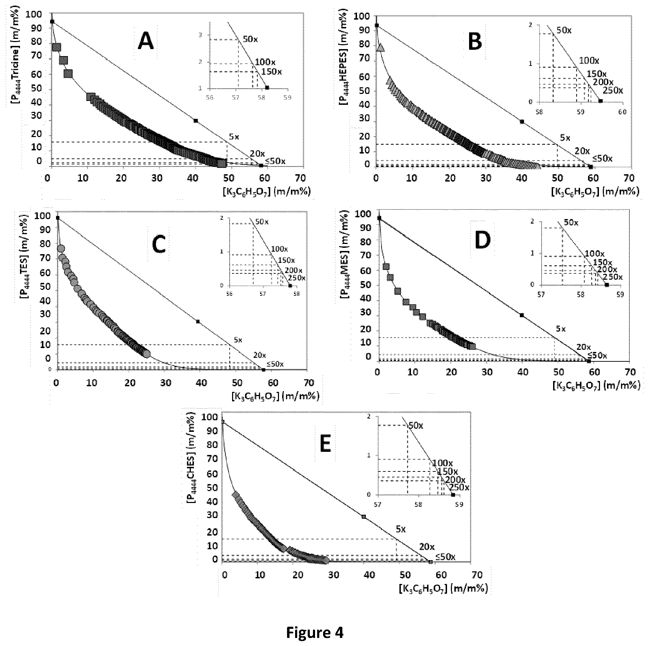

It is thus possible to conclude that the ABS proposed in this disclosure allow the extraction of PSA to the ionic liquid-enriched phase, and simultaneously concentrate PSA between 10 -1000-fold. The concentration factor is determined by the volume of the IL-enriched phase compared to the initial biological sample volume. This is achieved by the preparation of ternary mixtures with several compositions along the same equilibrium line and based in the lever arm rule, this information being obtained by the respective phase diagrams, which are described in **figure 4****.** Concentration factors may be predicted until infinity, the process, however, being limited by the weighing of the constituents and "possible" amounts of urine that may be obtained for a determined patient.

**Table 1:** Extraction efficiencies of PSA (EE_{PSA}%) at 25 °C in the systems composed of ILs and K3C6H5O7.

| **IL** | **Mass ratio (m/m %)** | | ***EE*_{PSA}%** |
|---|---|---|---|
| | **[IL]_{IL}** | **[salt]_{IL}** | |
| **[P₄₄₄₄][MES]** | **30.35 ± 0.15** | **30.84 ± 0.53** | **100** |
| | **30.01 ± 0.04** | **40.03 ± 0.14** | **100** |
| **[P₄₄₄₄][CHES]** | **30.13 ± 0.25** | **30.54 ± 0.63** | **100** |
| | **29.94 ± 0.13** | **40.12 ± 0.57** | **100** |
| **[P₄₄₄₄][HEPES]** | **30.16 ± 0.35** | **30.44 ± 0.29** | **100** |
| | **30.09 ± 0.34** | **39.97 ± 0.27** | **100** |
| **[P₄₄₄₄][Tricine]** | **30.90 ± 0.28** | **30.70 ± 0.31** | **100** |
| | **30.37 ± 0.17** | **39.98 ± 0.07** | **100** |
| **[P₄₄₄₄][TES]** | **29.95 ± 0.24** | **30.08 ± 0.69** | **100** |
| | **30.63 ± 0.37** | **39.85 ± 0.17** | **100** |

In an embodiment, the ABS composed of K₃C₆H₅O₇:[P₄₄₄₄][CHES]:urine in a mass ratio of 40%:30%:30% was the one that presented extraction efficiencies of 100%, but with the lower protein losses, as evidenced in **table 1** and **figure 3****,** and for that reason it was chosen for future assays. However, other mass ratios, namely 30%:30%:40% and other ionic liquids, namely [P₄₄₄₄][MES], [P₄₄₄₄][TES], [P₄₄₄₄][HEPES], [P₄₄₄₄][Tricine] were also tested.

In an embodiment, 30%:30%:40% and 40%:30%:30% ratios were tested for all ILs mentioned in the present disclosure.

In an embodiment, the nature of the interactions between the ionic liquids tested and PSA was evaluated by molecular docking studies that allowed to conclude that all anions from the tested ionic liquids (except for the [MES]⁻ anion) bind to amino acid residues that are far away from the PSA catalytic triad, therefore, not affecting, the catalytic activity thereof.

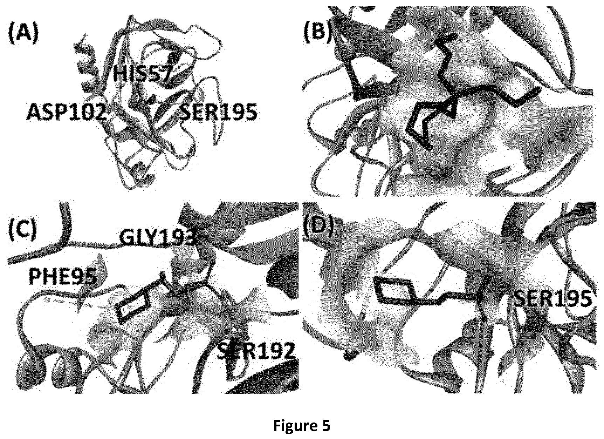

In an embodiment, the molecular docking studies also allowed to explain the high extraction efficiency of the systems studied; in the case of the ionic liquid [P₄₄₄₄][CHES] it was possible to verify that anion [CHES]⁻ binds to the zone adjacent to residues PHE95, SER192 and GLY193, not affecting the catalytic triad, as evidenced in **figure 5****.**

In an embodiment, the validation of the results with a real sample of urine was performed by a simple and widely available quantification method. The results obtained by size exclusion high-performance liquid chromatography (SE-HPLC) confirmed that in a real urine sample containing 150 ng/mL PSA, this biomarker is not quantifiable by SE-HPLC (no peak corresponding to PSA was observed in the chromatogram), as evidenced in **figure 6****.** However, after the application of the protocol described herein it is possible to detect PSA in the ionic liquid-enriched phase, the presence thereof not being observed in the other phase, which demonstrates the complete extraction and concentration of PSA in the ionic liquid-rich phase.

In an embodiment, the method may comprise the following steps:
- preparing an ABS by weighting of each of the system components, in particular urine, IL and salt. The amounts of each component to be used are determined from the solubility curve and from the equilibrium lines of the phase diagram of the ABS;
- homogenizing the system by vigorous stirring;
- centrifuging the system to promote phase separation;
- collecting the ionic liquid-rich phase where PSA and its isoforms are located.

In an embodiment, the ABS formed by the ionic liquids and salts proposed in this disclosure allow the selective extraction of PSA to the ionic liquid-enriched phase with extraction efficiencies of 100%. With these systems, it is also possible to concentrate PSA in the ionic liquid-rich phase between 10 - 1000-fold. Other than PSA, bPSA is also extracted, allowing advances in differential diagnosis.

In an embodiment, the disclosure now described was validated using real human urine. The results obtained by size exclusion high-performance liquid chromatography (SE-HPLC) confirmed that in a real sample to which 150 ng/mL PSA was added, this biomarker is not quantifiable by SE-HPLC (no peak corresponding to PSA was observed in the chromatogram). However, after the application of the protocol described herein it is possible to detect PSA in the IL-enriched phase, while the presence thereof in the salt-rich phase is not detected, demonstrating the extraction and concentration of PSA to one of the phases in a single step. The results allowed also to verify that together with PSA, bPSA is also extracted to the ionic liquid-rich phase.

In an embodiment, the pre-treatment process of human urine samples for the simultaneous extraction, purification and concentration of PSA may be performed in 4 steps: preparation of the liquid-liquid type system (ABS); homogenization; centrifugation; and phase separation with biomarkers being concentrated.

In an embodiment, the selective process for the extraction and concentration of PSA from urine is characterized by the addition of urine to a mixture of ionic liquid and salt.

In an embodiment, the ABS may be composed of ionic liquids from the imidazolium family, quaternary ammoniums and quaternary phosphoniums, with variable alkyl side chain size and presence of specific functional groups, combined with organic and inorganic anions, and sodium and potassium salts combined with organic and inorganic anions.

In an embodiment, the ABS contains ionic liquid in a concentration between 10 and 60 (m/m)% and salt between 5 and 50 (m/m)%, the ionic liquid being [P₄₄₄₄][CHES] and the salt being K₃C₆H₅O₇.

In an embodiment, the obtained mixture comprising the ionic liquid and the salt is centrifuged at a rotation speed between 187.80 - 18780xg, for a period of time between 1 to 60 minutes, at a temperature between 5 and 50 °C.

In an embodiment, the process now disclosed may comprise a step of recovering ionic liquid-enriched top phase from the aqueous biphasic system formed in the immediately previous step.

In an embodiment, the concentration between 10 - 1000-fold of PSA and its isoform (bPSA) in the ionic liquid-enriched phase allows the quantification thereof by a simpler less expensive and routine analytical method with higher detection limits.

### Example of application 1:

In an embodiment, the protocol for PSA quantification in urine samples may be performed as follows. This protocol may be directly applied to urine samples of patients for the quantification of PSA and use thereof in prostate cancer diagnosis.

In an embodiment, **figure 6** shows the chromatographic profile obtained from a urine sample without the application of the pre-treatment protocol proposed in this disclosure and of real urine samples to which the pre-treatment protocol has been applied.

In an embodiment, the profile illustrated in **figure 6(a)** serves as term of comparison and refers to the pure PSA aqueous solution. In this profile it is possible to observe the peak corresponding to the target biomarker, PSA, in minute 13.5. When a urine sample from a healthy donor is injected without any treatment it is not possible to observe PSA **(****Figure 6b****)** since its levels are very low and cannot be quantified by SE-HPLC. However, after the application of the protocol proposed in the present disclosure, the peak corresponding to PSA is clearly identifiable in the ionic liquid-rich top phase **(****Figure 6c****),** but it is not observable in the salt-rich bottom phase **(****Figure 6d****).** These results clearly demonstrate that PSA present in the urine of a healthy donor was extracted and concentrated to the IL-enriched phase, with a concentration factor that allows its quantification by HPLC. These results were reconfirmed by adding PSA to real urine, at a final concentration of 150 ng/mL **(****Figures 6e and 6f****).** Once more, PSA was extracted with an efficiency of 100% for the ionic liquid-rich phase, that is, totally and without any biomarker losses, with concentration factors between 10 - 1000-fold, allowing its quantification by SE-HPLC.

### Example of application 2:

In an embodiment, the extraction of PSA isoforms, in particular the extraction of bPSA, may be performed as follows. PSA is a glycoprotein which may be found in different isoforms, and the levels of those different forms and respective proportions may be used in the differential diagnosis of prostate cancer. From the analysis of **figure 6** it is possible to observe the existence of a peak of lower intensity between minutes 16 and 17, being identified as bPSA. Therefore, the present disclosure is able not only to concentrate PSA, but also bPSA, opening new perspectives to the use of this technology in the differential diagnosis of prostate cancer.

Even though in the present disclosure only particular embodiments thereof are represented and described, a person skilled in the art will know how to introduce modifications and substitute technical features with other equivalents, depending on the requirements of each case, without leaving the scope of protection defined by the appended claims. The embodiments presented are combinable with each other. The following claims define additional embodiments.

### References

(1) Ferley et al., International Journal of Cancer, 2015, 136, E359-E386.
(2) Luengo-Fernandez et al., The Lancet Oncology, 2013, 14, 1165-1174.
(3) Bolduc et al., Canadian Urological Association Journal, 2007, 1(4), 377-381.
(4) Fedotoff et al., Journal of Biomolecular Structure and Dynamics, 2012, 29, 1051-1064.
(5) Klein et al., European Urology, 2017, 72(6), 942-949.
(6) Taha et al., Green Chemistry, 2014, 16, 3149-3159.

## Claims

1. Process for extracting, concentrating and purifying a prostate-specific antigen (PSA) and/or a benign prostate-specific antigen (bPSA) from a biological sample, comprising the following steps:
extracting the prostate-specific antigen (PSA) or benign prostate-specific antigen (bPSA) by the preparation and homogenization of an aqueous biphasic solution comprising the biological sample with an ionic liquid and a salt;
concentrating said homogenized solution; and
purifying the prostate-specific antigen (PSA) and/or benign prostate-specific antigen (bPSA) of said concentrated solution.

2. Process according to the preceding claim, wherein the mass ratio between the ionic liquid and the biphasic solution ranges between 10-60 % (m_{ionic liquid/}m_{aqueous biphasic solution}), preferably a mass ratio of 20-50 % (m_{ionic liquid}/m_{aqueous biphasic solution}), even more preferably a mass ratio of 30% (m_{ionic liquid/}m_{aqueous biphasic solution}).

3. Process according to any of the preceding claims, wherein the ionic liquid is selected from the following list: tetrabutylphosphonium cyclohexyl-2-aminoethanesulfonate ([P₄₄₄₄][CHES]), tetrabutylphosphonium 2-(N-morpholino)ethanesulfonate ([P₄₄₄₄][MES]), tetrabutylphosphonium 2-[[1,3-dihydroxy-2-(hydroxymethyl)propan-2-yl]amino]ethanesulfonate ([P₄₄₄₄][TES]), tetrabutylphosphonium 2-[4-(2-hydroxyethyl)piperazin-1-yl]ethanesulfonate ([P₄₄₄₄][HEPES]) or tetrabutylphosphonium N-2(2-hydroxy-1,1-bis(hydroxymethyl)ethyl)glycine ([P₄₄₄₄][Tricine]), preferably wherein the ionic liquid is tetrabutylphosphonium cyclohexyl-2-aminoethanesulfonate ([P₄₄₄₄][CHES]).

4. Process according to any of the preceding claims, wherein the mass ratio between the salt and the biphasic solution ranges between 5-50 % (m_{salt/}m_{aqueous biphasic solution}), preferably between 10-45 % (mₛₐₗₜ/m_{aqueous biphasic solution}), even more preferably it is of 40% (m_{salt/}m_{aqueous biphasic solution}).

5. Process according to any of the preceding claims, wherein the salt is selected from the following list: sodium salt combined with organic or inorganic anions or potassium salt combined with organic or inorganic anions.

6. Process according to any of the preceding claims, wherein the salt is potassium citrate (K₃C₆H₅O₇) or sodium citrate (Na₃C₆H₅O₇).

7. Process according to any of the preceding claims, wherein the ionic liquid is tetrabutylphosphonium cyclohexyl-2-aminoethanesulfonate ([P₄₄₄₄][CHES]) and the salt is potassium citrate (K₃C₆H₅O₇).

8. Process according to any of the preceding claims, wherein the concentrating step comprises concentrating the prostate-specific antigen and benign prostate-specific antigen (bPSA) 10-1000-fold regarding the concentration of the prostate-specific antigen and benign prostate-specific antigen (bPSA) in the biological sample, preferably urine.

9. Process according to any of the preceding claims, wherein the step of concentrating the prostate-specific antigen and benign prostate-specific antigen (bPSA) is performed by centrifugation, preferably at 187.80 - 18780 xg.

10. Process according to any of the preceding claims, wherein the purifying step is performed by extraction of the prostate-specific antigen (PSA) and benign prostate-specific antigen (bPSA) to an ionic liquid-enriched phase after the purifying step.

11. Process according to any of the preceding claims, wherein the biological sample has a mass ratio between 5-40 % (m_{biological sample}/m_{aqueous biphasic solution}), preferably has a mass ratio of 10-35 % (mb_{iological sample}/m_{aqueous biphasic solution}), even more preferably has a mass ratio of 30% (m_{biological sample}/m_{aqueous biphasic solution}).

12. Process according to any of the preceding claims, wherein the aqueous biphasic solution has the following salt: ionic liquid: biological sample mass ratio of 40:30:30 (mₛₐₗₜ:m_{ionic liquid}:m_{biological sample}).

13. Process according to any of the preceding claims, wherein the biological sample is urine, in particular is human urine.

14. Process according to any of the preceding claims, wherein the purifying step is performed between 1-60 minutes, preferably between 10-50 min, even more preferably between 20-40 min.

15. Process according to any of the claims, wherein the purifying step occurs at a temperature between 5-50 °C, preferably between 10-30 °C, even more preferably between 18-25 °C.

16. Process according to any of the claims, wherein the extracting step is performed by means of a magnetic stirrer.

17. Composition comprising a biological sample, an ionic liquid and a salt, for use in medicine.

18. Composition comprising a biological sample, an ionic liquid and a salt, for use in an "in vivo" diagnostic method of the prostate cancer disease.

19. Composition for use according to any of claims 17-18, wherein the ionic liquid has a mass ratio between 10-60 % (m_{ionic liquid/}m_{aqueous biphasic solution}), preferably has a mass ratio of 20-50 % (m_{ionic liquid}/m_{aqueous biphasic solution}), even more preferably has a mass ratio of 30% (m_{ionic liquid}/m_{aqueous biphasic solution}).

20. Composition for use according to any of claims 17-19, wherein the ionic liquid is selected from the following list: tetrabutylphosphonium cyclohexyl-2-aminoethanesulfonate ([P₄₄₄₄][CHES]), tetrabutylphosphonium 2-(N-morpholino) ethanesulfonate of ([P₄₄₄₄][MES]), tetrabutylphosphonium 2-[[1,3-dihydroxy-2-(hydroxymethyl)propan-2-yl]amino]ethanesulfonate ([P₄₄₄₄][TES]), tetrabutylphosphonium 2-[4-(2-hydroxyethyl)piperazin-1-yl]ethanesulfonate ([P₄₄₄₄][HEPES]) or tetrabutylphosphonium N-2(2-hydroxy-1,1-bis(hydroxymethyl)ethyl)glycine ([P₄₄₄₄][Tricine]), preferably wherein the ionic liquid is tetrabutylphosphonium cyclohexyl-2-aminoethanesulfonate ([P_{4444][}CHES]).

21. Composition for use according to any of claims 17-20, wherein the salt has a mass ratio between 5-50 % (m_{salt/}m_{aqueous biphasic solution}), preferably has a mass ratio of 10-45 % (m_{salt/}m_{aqueous biphasic solution}), even more preferably has a mass ratio of 40% (mₛₐₗₜ/m_{aqueous biphasic solution}).

22. Composition for use according to any of claims 17-21, wherein the salt is potassium citrate (K₃C₆H₅O₇) or sodium citrate (Na₃C₆H₅O₇).

23. Composition for use according to any of claims 17-22, wherein the ionic liquid is tetrabutylphosphonium cyclohexyl-2-aminoethanesulfonate ([P₄₄₄₄][CHES]) and the salt is potassium citrate (K₃C₆H₅O₇).

24. Composition for use according to any of claims 17-23, wherein the biological sample has a mass ratio between 5-40 % (m_{biological sample}/m_{aqueous biphasic solution}), preferably has a mass ratio of 10-35 % (m_{biological sample}/_{maqueous biphasic solution}), even more preferably has a mass ratio of 30% (m_{biological sample}/m_{aqueous biphasic solution}).

25. Composition for use according to any of claims 17-24, wherein the biological sample is urine, in particular is human urine.

26. Composition for use according to any of claims 17-25, wherein the composition comprises a salt:ionic liquid:biological sample mass ratio of 40:30:30 (mₛₐₗₜ:m_{ionic liquid}:m_{biological sample}).

27. Kit comprising the composition according to any of claims 17-26.
